# EUROPEAN PATENT SPECIFICATION

(11) **EP 0 546 222 B1**
(45) Date of publication and mention of the grant of the patent: **10.09.1997**
(21) Application number: 91308968.6
(22) Date of filing: 01.10.1991
(51) Int. Cl.: G01N 33/543, G01N 33/58, G01N 33/569, G01N 21/77

(54) **Highly sensitive optical immunoassay using enzyme-labeled reagents**
Hochempfindlicher optischer Immunoassay durch Gebrauch von Enzymmarkierten Reagenz
Immunoessai optique à haute sensibilité utilisant des réactifs marqués avec des enzymes

(43) Date of publication of application: 16.06.1993
(73) Proprietor: BIOSTAR, INC., Boulder, CO 80301 (US)
(72) Inventor: Maul, Diana M., Thornton, Colorado 80221 (US); Crider, Debbie G., Boulder, Colorado 80301 (US); Bilodeau, Robert J., Arvada, Colorado 80005 (US); Bogart, Gregory R., Ft. Collins, Colorado 80521 (US)
(74) Representative: Sexton, Jane Helen

(56) References cited:
- EP-A- 0 089 939
- EP-A- 0 322 549
- EP-A- 0 347 138
- WO-A-88/04932
- WO-A-89/09937
- WO-A-90/11525
- WO-A-91/04491
- WO-A-91/05261
- WO-A-91/06862
- WO-A-92/03730
- FR-A- 2 191 744
- US-A- 4 332 476
- SPIE, Vol. 954 Optical Testing and Metrology II (1988), pages 413-419

## Description

### FIELD OF THE INVENTION

This invention relates to highly sensitive, thin film, immunoassay (OIA) devices and a process for detecting small amounts of substances derived from analytes of interest by enzyme-labeled means.

Immunoassays based on thin film optical effects have a long history dating back to the work of Giaever. Such assays depend on the increase in thickness at a surface giving an optical effect visible to the eye or an instrument, including but not necessarily, as a color change. Sometimes the analyte of interest may be sufficiently large to cause itself a significant observable thickness change when it binds to the optically suitable surface. However, many analytes of interest are more readily detected when an additional particulate reagent is added to the assay giving a greater final thickness change. The particles chosen for this purpose have been polystyrene latexes which can readily adsorb antibody to their surface and, hence, function in the amplifying detection of the corresponding antigen. Such polymeric latexes are available in a range of sizes, but for practical purposes, their utility in the assay is a compromise between size, which gives increased thickness, and surface area, which contributes to sensitivity; smaller particles give less increased thickness; larger particles have less surface area for binding antibody. Thus, particulate reagent enhancers used in optical thin film immunoassays have physical limitations which impose constraints of sensitivity on the assay and limit the applicability of such assays in the measurement of clinically important analytes of interest.

This invention overcomes the limitations imposed by the prior art use of particulate reagent enhancers. By the use of antibody-enzyme conjugates in place of latex-reagent particles it has surprisingly been found that even more highly sensitive optical thin film assays can be obtained, particularly with selected substrates for the enzyme which provide insoluble precipitated products. The present invention relates to the use of such enzyme-labeled antibody methods in thin film assays for the detection of low levels of the polysaccharide antigens derived from the group of bacteria commonly responsible for bacterial infections in man, such as meningitis and streptococcus. The detection and identification of such antigens is important in the diagnosis and effective choice of treatment for these significant bacteria originated disorders.

### BACKGROUND OF THE INVENTION

### Description of the Prior Art

It is known that various diagnostic systems and elements useful in optical immunoassay techniques rely upon latex-based particles, some of which need to employ enhancing substances. By some of these methods, the agglutination immunoassay relies on the antibody to produce particles of sufficient size, through aggregation, to generate a visible signal. The visualization is achieved by light scattering. In other methods, the highly crosslinked, rigid co-polymers provide solid support for the capture and separation of the analyte of interest. It is also known that enzyme-linked immuno-sorbent assays (ELISA's) are both well accepted and widely utilized for both qualitative and quantitative assays for biomolecules. Various investigators have utilized the process of linking an enzyme to an antibody against the molecule to be measured, that is, the analyte of interest, and then used it in combination with a chromogenic substrate specific for that enzyme which yields a visible colored reaction product. For those processes, test tubes or filter membranes are used as the solid phase on which the molecule to be measured is immobilized. More commonly, in the membrane based assays, the secondary antibody is attached to dyed latex or a metallic particle for color generation. Such reactions involving color changes are found in J. Immunoassay, 2 (3 & 4), 187-204, 1981. Once such a colored product is obtained, it can be qualitatively observed or quantitatively measured by various techniques, including spectrophotometry. While the number of variations on such assay techniques are numerous, they all depend solely on the rate and extent of color formation as proportionally related to the presence and concentration of antigen.

The formation of optically detectable color records of results of ELISAs using various enzymes such as horseradish peroxidase (HRP) is disclosed in Woiszwillo, U.S. Patent 5,013,646. Also, Bishop et al, U.S. Patent 5,024,935, discloses utilizing a peroxidase labeled specific binding compound in the form of an antibody against human chorionic gonadotropin (HCG) to measure the amount of dye present as an indication of the amount of ligand present. In McClune, U.S. Patent 5,017,474, the ligand is determined by methods which involve formation and detection of an immunological complex of ligand and receptor in a peroxidase-reactive system which yields a dye. Similar methods are disclosed in Satoshi et al, U.S. Patent 4,921,791. In Tung, U.S. Patent 4,788,138, a horseradish peroxidase enzyme is used as an antibody label which binds to an insoluble support through a complex formed with the antigen and at least two antibodies.

WO 91/05261 discloses a biosensor detector method for detecting biological targets, using specific binding, or hybridization techniques coupled with enzymatic amplification and the mass sensing capability of a piezoelectric oscillator.

WO 89/09937 discloses a quartz crystal microbalance assay in which the binding of analyte to a surface on or near a quartz crystal microbalance (QCM) is detected by a conjugate which comprises an enzyme capable of catalyzing the conversion of a substrate to a product capable of accumulating on or reacting with a surface of the QCM leading to a mass change and, hence, a change in the resonant frequency.

WO 90/11525 discloses an assay for an analyte, particularly a hapten, where changes in refractive index at a solid surface are monitored by means of surface plasmon resonance spectroscopy. The signal is enhanced by conjugating to an assay reagent a substance capable of giving a strong signal.

WO 91/06862 discloses that layers of transparent dielectric material having immobilised thereon haptens or hapten analogues are useful in devices for the immunoassay of haptens which are based on the principle of frustrated reflection the layer of transparent dielectric material forms part of a biosensor comprising (a) a cavity layer of transparent dielectric material having immobilised upon it a hapten or hapten analogue, (b) a dielectric substrate, and (c) interposed between the cavity layer and the substrate, a dielectric spacer layer, the arrangement being such that, when the interface between the substrate and the spacer layer is irradiated with light such that total reflection occurs, the total reflection may be frustrated by the propagation of a resonant guided mode within the cavity layer.

WO 92/03730, which forms part of the state of the art under Article 54(3) and (4) EPC, discloses a biosensor for the detection of an analyte in solution using enzyme-substrate interaction for response amplification. The biosensor device may be based on the principle of frustrated total reflection.

It is an object of the present invention to provide an improved optical immunoassay system with greater sensitivity than exhibited by latex particle agglutination surface systems.

It is still another object to provide for a highly sensitive, dependable, accurate and simple optical immunoassay system for detecting the presence of infectious bacterial analytes such as Neisseria meningitidis A, C, Y, W₁₃₅, and the like.

It is yet another object of the present invention to utilize enzyme substrates and enzyme-labeled antibodies capable of providing insoluble, detectable optical thickness changes on a non-particulate, non-latex substrate surface in the presence of even very low levels of specific antigens.

These and other objects have been achieved by the present invention as disclosed hereinafter more fully.

Accordingly, the invention provides a method as defined in Claim 1, and an optical assay device as defined in Claim 12, for detecting an analyte of interest.

The present invention includes novel compositions and novel methods using an article for the direct selective binding, by whatever mechanism, of the analyte of interest from serum or other bodily fluid, such as cerebrospinal fluid (CSF) or various substances for purposes of identification. The present invention utilizes, in its broadest sense, a non-polymeric, non-particulate, non-latex substrate coated with one or more layers including an unlabeled receptive material. This material has a highly receptive function and is adapted to directly interact with a substance of the analyte of interest and an enzyme-labeled secondary receptive material. This secondary receptive material may or may not be identical to the unlabeled receptive material immobilized on the solid support. Such a substance can be a polysaccharide indicative of a surface antigen or cell component of a bacteria such as various species of streptococcus, Neisseria meningitidis, Haemophilus, and the like.

### BRIEF DESCRIPTION OF THE DRAWINGS

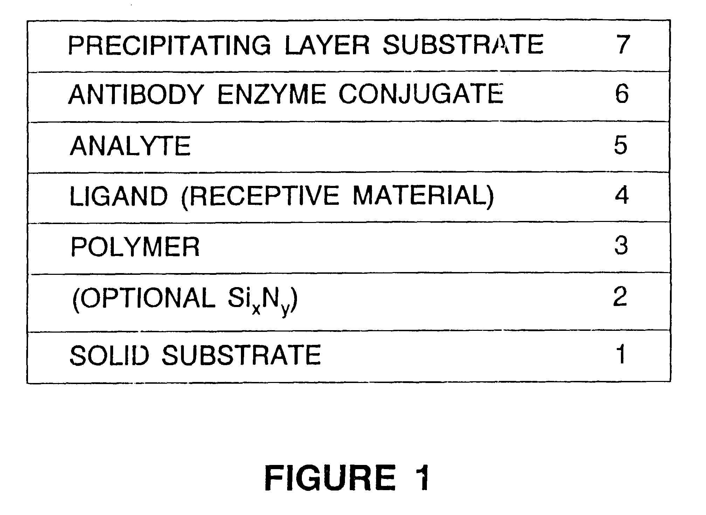

Figure 1 represents a longitudinal cross-section of the multilayer, thin film optical immunoassay device depicting the various layers including those of the ligand layer, the enzyme-labeled antibody layer and the topmost substrate comprising the precipitating agent.

### DETAILED DESCRIPTION OF THE PREFERRED EMBODIMENTS

Figure 1 is a graphic representation of cross-section of the multilayer OIA device comprising a substrate 1 having an upper and lower surface and upon whose upper surface, various layers are mediately and directly coated. These layers include an optional layer of silicon nitride immediately adjacent that upper layer, as more fully disclosed in co-pending application WO 91/04491. If that layer is present, over it or in its absence, over the upper layer, there is coated or deposited a polymer layer 3, such as a polymeric siloxane. This polymer layer provides an intermediate surface modification layer with an improved protein adhesion and environment which supports the ligand layer 4. This polymer layer must be sufficiently thick to insulate the supported biomolecule layers from whatever toxic effects the substrate may offer. The ligand layer is one member of a binding pair, such pairs include, but are not limited to, antigen-antibody, oligonucleotide/DNA chelator/metal, enzyme-inhibitor, enzyme-substrate, bacteria-receptor, virus-receptor, hormone-receptor, DNA/RNA or RNA/RNA and any other binding or reactor combination of species. The ligand layer or receptive material layer supports an analyte layer 5 which in turn bears a top-most interactive layer of antibody-labeled enzyme 6, such as an antibody specific for the antigen which is, or may be, derived from the analyte of interest. When so combined, and complexed, the enzyme-labeled antibody and analyte layers are simultaneously a complexed solid, defined conjugate mass. It is over this mass that the top-most covering or substrate 7, such as TMBlue, is added dropwise to cause the precipitate described.

It is an embodiment of the present invention that the processes, devices and kits embodying the present invention include analytes of interest, such as viral antigens, DNA, RNA, hormones, and any other similar analyte.

Table 3 of Example 3 is represented by a graph comparing immunoassay devices of the prior art, i.e., latex-particle reagent derived and the enzyme-labeled OIA devices of this invention. It will be seen that as the dilution decreases for antigens derived from the bacterial analyte of interest, there is a marked inability of the latex reagent device to provide sufficient signal based on the optical thickness of the antigen-antibody-latex layer. In contrast, enzyme-labeled antibody-antigen complexes of the present device produce at least a 5x increase in measurable sensitivity. Typical of the enzymes useful in the present invention are glucose oxidase, galactosidase peroxidase, alkaline phosphatase and the like. It will be noted that the enzyme reagent provides an unstable thickness increase when highly concentrated antigen is present. This is not mechanically stable and is removed from the surface easily by rinsing and drying. This causes an apparent saturation in signal which is merely an artifact of the precipitated product. However, a strong blue color is generated in the sample spot prior to removal of excess fluid; indicating a positive result.

In one embodiment of the present invention, there is provided a highly sensitive thin film optical immunoassay device comprising a substrate having an upper and a lower surface and supporting mediately on its upper surface, at least one layer comprising an immobilized peroxidative enzyme conjugate capable of interacting with a peroxide-delivery reactive substance derived from an analyte of interest to form an insoluble reaction product. This insoluble reaction product comprising immobilized antibody-antigen-antibody HRP Complex is precipitated by the action of a precipitating agent such as TMBlue, 3,3',5,5'-tetramethylbenzidene, present as a substrate contacting the reaction product layer when in combination of alginic acid, dextran sulfate, methyl vinyl ether/maleic anhydride copolymer, or carrageenan and the like. Other substances such as chloro-napthol, diaminobenzidene tetrahydrochloride, aminoethyl-carbazole, orthophenylenediamine and the like can also be used. These are used in concentrations from about 10 to about 100 mM. It is by these means that a measurable increase in optical thickness occurs on said peroxidative enzyme conjugate layer and the unlabelled antibody layer. This change is unaffected by and not at all dependent on color formation of the peroxide-delivery reactive substance.

In sequential fashion, once the antigen, either derived from the analyte of interest, or the analyte of interest directly reacts with the labeled antibody in solution, it results in the antigen-labeled antibody complex which in turn reacts with the unlabeled antibody bound to the non-latex, non-polymeric, non-particulate support.

In another embodiment there is provided a process for detecting the presence of an analyte of interest by the steps of providing the device above-described, interacting said device with a peroxide delivery reactive substance derived from said analyte of interest, and forming an insoluble reaction product. This is then washed and dried in order to separate, discard and remove any unreacted layer material, then the change in optical thickness is measured and recorded and the increase of the peroxidative enzyme conjugate layer determined by various means including a visual means or by the use of instrumentation, such as ellipsometry and the light intensity differentials caused by the increased thickness. The receptive enzyme material is thus capable of direct interaction with the analyte of interest and more particularly evidence of such analyte, such as an antigen, so that a visible change in the thin film is obtained. This change is detectable by measuring the optical thickness and does not necessarily depend on any light reflectivity of the substrate material. One such instrument above-noted is the Sagax Ellipsometer, disclosed in U.S. Patent 4,332,476 along with U.S. Patents 4,655,595, 4,647,207, and 4,558,012.

Materials which can be included in the overall class of unlabeled receptive materials includes toxins, antibodies, antigens, hormone receptors, parasites, cells, haptens, metabolites, allergens, nucleic acids, nuclear materials, autoantibodies, blood proteins, cellular debris, enzymes, tissue proteins, enzyme substrates, coenzymes, and neuron transmitters, viruses, viral particles, microorganisms, bacteria, metals, and various chemical species and materials derived therefrom. This list incorporates only some of the many different materials that can be coated onto the coated substrate surface to produce an optical immunoassay system with an enzyme-labeled secondary receptive material. Whatever the selected analyte of interest is, the receptive material is designed to interact specifically with that analyte of interest. By the use of the term "enzyme-labeled", it will be understood to mean an enzyme which is conjugated or otherwise specifically prepared to be received, such as an antibody-enzyme conjugate. The enzyme-labeled secondary receptive material is only captured on the immobilized unlabeled receptive material in the presence of antigen.

Although the examples use a silicon wafer as a substrate, it is contemplated that a variety of substrate material can be employed. The substrate can be formed from a silicon crystal which is diamond sawed to form a wafer which is then subjected to an anisotropic etch in KOH, and then is isotropically etched to form a smoother surface. One surface of such a wafer is polished producing a smooth mirror-like finish with a specularly reflecting surface. The reverse surface remains slightly irregular with ridges and valleys on the order of 200-300 nm in height producing a diffusely reflecting surface. Either side of the wafer can be employed, although the specular side is the preferred for instrumented detection. Silicon wafers that are rejected by the semiconductor industry can be employed in this invention, thus reducing assay manufacturing costs. Level and type of dopant in the silicon wafer are irrelevant to this invention.

As shown in Figure 1, the substrate has an upper surface that is adapted to support additional material. This upper surface has the characteristics necessary for production of a signal. The intermediate layer material is any material that produces a favorable microenvironment for the receptive material and which allows for the eventual production of a dense viable layer of receptive material.

The intermediate layer may be produced by application of one or more materials which will adhere to the substrate through different mechanisms. The siloxanes covalently modify the substrate while the other materials simply adhere to the surface, although without any subsequent delamination and are stable to most mechanical manipulation. Methods of coating polymers to substrates are known to those skilled in the art of semiconductors. It is contemplated that the polymeric materials could be attached by spin coating, aerosol spraying, dipping, etc. The method of coating can be tailored to the type of the intermediate layer material employed.

Although not required, additional materials which convey a desired property may be affixed to the intermediate coated substrate. Such a layer could improve receptive material orientation as in the use of Protein A or Protein G for orienting antibodies. Other materials which could be utilized include avidin-biotin, synthetic or recombinant peptides, etc.

In one preferred embodiment, the intermediate material is spin coated or aerosol spray coated in a uniform manner. The various intermediate materials, when coated to the substrate at thicknesses between 0.5 and 50 nm (5 Angstroms and 500 Angstroms) (thicker amounts can be employed), provide the assay test surface with the advantages previously listed. The layer can be formed of any material that performs the following functions and has the following characteristics: creates a favorable environment for the receptive material; permits the receptive material to be densely bound in active positions by a cost-effective method; exhibits low non-specific interactions; adheres covalently or tightly to the substrate; and can be coated to the substrate uniformly but not necessarily continuously. The intermediate layer material can be placed on the substrate in various ways.

As shown in Figure 1, the intermediate layer material has a lower surface which is attached to the upper substrate surface. This lower surface is adapted to be compatible with the substrate. The polymeric layer material also has an upper polymer surface that is adapted to adhere the interactive, immobilized receptive material. The intermediate coated test surface is usually stable and can be stored prior to coating with receptive material.

After the intermediate layer material is coated to the substrate it may require a curing period. It has been noted that the T-polymer siloxane works best when it is given a period of time in which to cure prior to application of the chosen receptive interactive species.

The immobilization chemistry for attaching the receptive material to the intermediate layer material is selected based on the properties of both the coated substrate and the receptive material. The receptive material can be covalently or passively attached to the intermediate layer material.

It will be understood by those skilled in the art that the stability of antibody-coated wafers can be enhanced by various protectants, such as, for example, gelatin hydrolysates and the like. One of the problems to be overcome is obtaining a uniformity of protective overcoating and the regularity of the spreadability of the coating substance. It has been found that good results are obtained by washing off the protective coating, then air-drying the surface before the immunoassay is done, although some circular spotting may remain. However, since the assay is not spot or color dependent, this is relatively unimportant.

### EXAMPLE 1

### Description of the Reagents and Assay Method

Horseradish peroxidase (Sigma grade VI) was chemically coupled to immunoglobulins purified by caprylic acid precipitation from pooled high titer sera from rabbits previously injected with suspensions of cells from cultures of Neisseria meningitidis A,C,Y W₁₃₅. The coupling was done using the reagent S-acetyl thioacetic acid N-hydroxysuccinimide ester and methods described in Analytical Biochemistry 132 (1983) 68-73. The resultant conjugate contained peroxidase (104µM) and immunoglobulin (35µM) in a buffer of MOPS, 50 mM, pH 7.0. The peroxidase-immunoglobulin conjugate was diluted in MOPS buffer together with casein (5 mg/ml) and mixed with an equal volume of a dilution of a cell-free filtrate from a culture of Neisseria meningitidis organisms. The mixture (25µl) was pipetted to the surface of a silicon wafer already coated with layers of silicon nitride, t-polymer siloxane and purified immunoglobulin from the same rabbit antiserum to Neisseria meningitidis. Antibody was coated to the t-polymer/silicon wafer from a solution containing 10µg/ml of antibody in 50 mM MOPS, pH = 7.0. The wafer remained in the antibody for 1 hour at ambient temperature, was rinsed with deionized water, and dried under a stream of nitrogen. The antibody coated substrate was further treated by incubating the coated substrate in 0.5 mg/ml hydrolyzed casein in 50 mM MOPS pH = 7.0 for 1 hour at ambient temperature followed by rinsing and drying as previously described. After 2 minutes the drop was washed off with water and the wafer was dried with a current of nitrogen or blotted with a filter device. TMBlue precipitating substrate (TMBlue is a commercially available product, trademarked by Transgenic Sciences, Inc. and disclosed in U.S. Patent 5,013,646) was applied to the same area of the wafer and allowed to stand for 5 minutes. The wafer was washed and dried. A purple spot was visible where the reaction had occurred. This resulting precipitate was then read by eye and ellipsometer to confirm the presence of N. meningitidis.

**Table 1**

| N. Meningitidis Results | | |
|---|---|---|
| FOLD DILUTION* | VISUAL SCORE | mVOLTS |
| 0 | - | 64.2 |
| 1:10,000 | + | 152.0 |
| 1:5,000 | +++ | 238.5 |
| 1:2,500 | +++ | 395.7 |
| 1:1,000 | ++++ | 635.0 |

| | | |
|---|---|---|
| *Dilution of the stock antigen preparation into 50 mM MOPS. Visually a 1:20,000 dilution of the antigen is clearly resolved from the negative. The Wellcogen kit's sensitivity cut-off is a 1+ at a 1:4,000 dilution of the same antigen preparation. | | |

### Test Kit

The test kit contains all the components necessary to perform up to 50 optical immunoassay rapid tests. The kit features a solid support test station which is designed to facilitate the proper washing and drying steps required. A slide, which may include from one to five unreacted test surfaces specific for the conjugated analyte of interest, is placed on the test station. Upon completion of the first reaction, the slide is tilted forward away from the operator. The test surface(s) are vigorously rinsed with wash solution which drains from the tilted surface into the reservoir below. The reservoir contains a solid absorbent block of cellulose acetate treated with biocide. The slide is then returned to a level position, and a piece of absorbent paper is placed directly onto the test surface. Several seconds contact time is allowed to give full wicking. The absorbent papers are provided as pads of individual tear-off sheets conveniently located on the front of the test kit, but the wash/dry process can be effected by alternate means, such as capillary action. In addition, a solution of an enzyme-labeled substance, an enzyme-labeled antibody which is specific to an analyte of interest (such as an antigen) is also provided, suitably buffered and diluted. Finally, precipitating means, such as a container of commercially available TMBlue liquid, is provided in a convenient volume so that one to three drops or more can be applied dropwise to cause the mass change to precipitate before washing. The second incubation is started by adding substrate to the surface and the wash/dry process is repeated to complete the test.

### EXAMPLE 2

Two different types of silicon wafers were used; one was a gold silicon nitride-coated wafer and the other was a silver-colored silicon wafer without a nitride coating. One possibility is that the visual color which is observed with the peroxidase/precipitating substrate system on the silicon nitride is strictly due to the absorbance of the dye precipitated on the surface. If this is the case and the precipitated dye is not behaving as a thin film, then the silver-colored silicon wafer will generate a visual signal which is the deep blue of the TMBlue only.

T-polymer coated wafers were treated with production grade antibodies which are used in the commercially available Wellcogen (trademark of Wellcome Diagnostics) latex agglutination tests. Reagents for five separate tests; N. meningitidis A,C,Y, W₁₃₅; N. meningitidis B; Streptococcus B; H. influenza; and Streptococcus pneumoniae were produced. The first reagent produced for each test was wafers, gold and silver, coated with the intermediate layer (T-polymeric siloxane) as previously described. All five antibodies were coated to these types of wafers at a 10µg/ml concentration of antibody in 50 mM MOPS, pH = 7.0 by immersing wafers in the appropriate solution for one hour at ambient temperature. Wafers were rinsed, dried, and blocked as described in Example 1.

The second reagent required utilized the same five antibodies for the production of antibody-horseradish peroxidase conjugates as described in Example 1. The stock conjugate preparations are used to product working conjugate by dilution in 50 mM MOPS, pH = 7.0, 5 mg/ml casein, to a final conjugate ratio of 1:100. One part of the working conjugate solution is mixed with one part of a standard antigen preparation, and a 20µl sample applied to the appropriate antibody coated wafers.

A rapid protocol was employed using a 2 minute first immune incubation followed by a wash, dry then a 5 minute substrate incubation to permit the build-up of product on the wafer surface. Following a wash and blot dry, the sample was read with both the naked eye and an ellipsometer. Purple colored spots, strikingly visible, developed on the gold, silicon nitride-coated wafer, and grey spots were seen using the silver-colored silicon wafer without nitride coating. The thickness increase could be readily measured using the ellipsometer. The visible color generated, in all cases, on the silver wafers, indicate that the precipitated product behaves as a true thin film and generates an interference effect even in the absence of an anti-reflective coating. The color generated is not dependent on the dye's absorbance characteristics.

Further evidence that the chromogen does not contribute to the generation of the observed visual response was gained with the following experiment. Treatment of the TMB/H₂O₂ product with a stopping reagent, H₂O₄, produces a yellow precipitate. If the visual response observed with the optical supports under investigation here is solely due to the chromogen, then the treatment of the surface precipitate with stopping reagent should yield a yellow-colored spot. Treatment of the immobilized surface precipitate with sulfuric acid does not modify the strong purple or blue spot generated on the silicon nitride coated wafer. Therefore, the resultant signal is entirely dependent on the formation of a thin film. Additional verification was obtained by using a strip of adhesive to remove the precipitate from the surface of the silicon nitride. The precipitate removed with the adhesive was a pale grey/blue with no red component. The observed interference effect exhibits a bright purple/blue color with a strong red component. This re-enforces that thin film formation is responsible for the generation of the observed color effect.

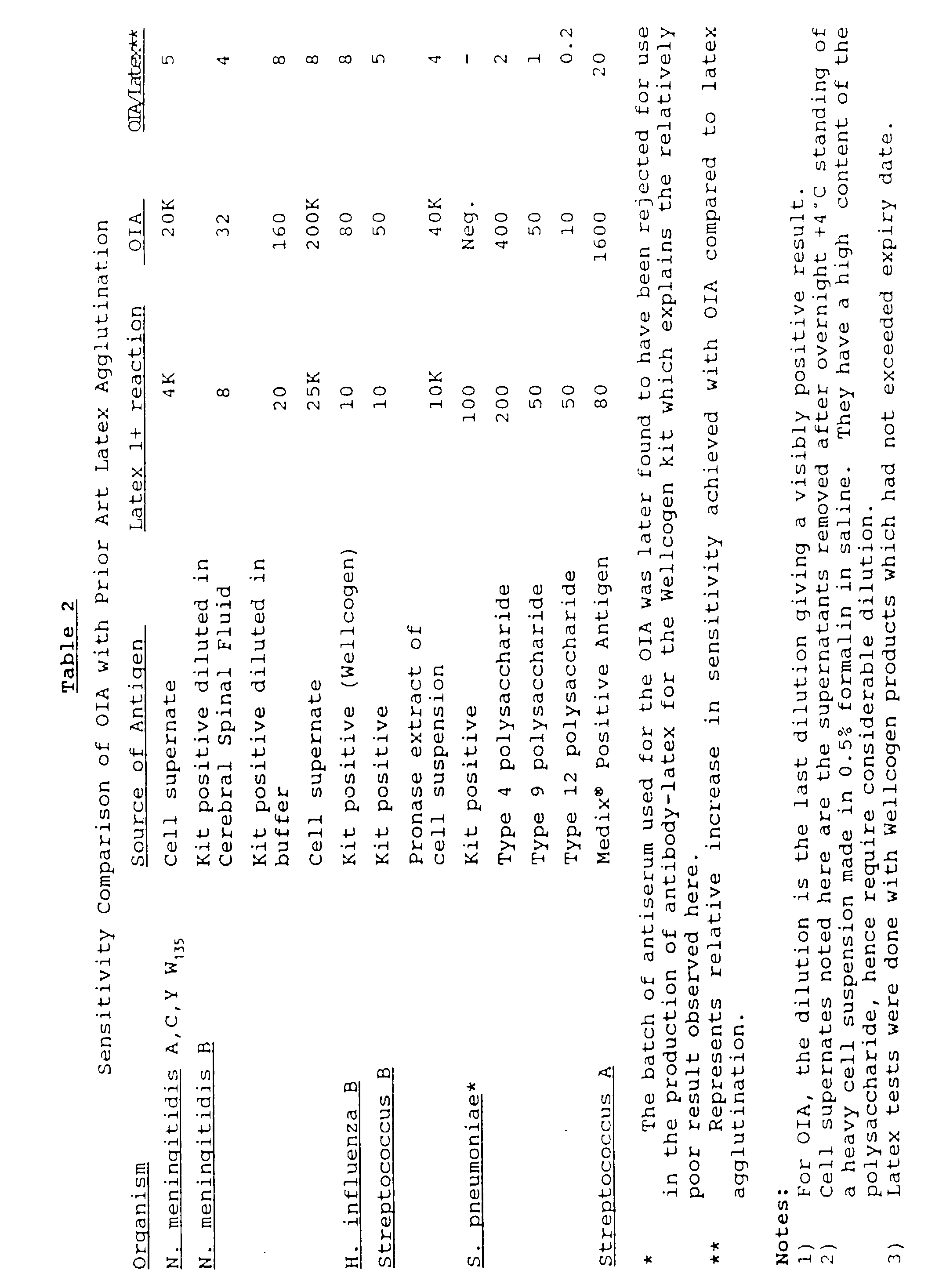

### EXAMPLE 3

### Comparison with Latex Particle Enhanced Optical Immunoassay

The new enzyme-labeled assay was used to detect antigen from Streptococcus A and compared on the same silicon wafer with an assay using amide modified surface activator latex, 0.161µm (Rhone-Poulenc). The assay using the enzyme-antibody was 5x more sensitive than that using the latex-antibody.

The increase in sensitivity was based on visual resolution of a 1:1600 dilution of the antigen control in the enzyme technique versus a 1:320 dilution for the latex based OIA technique. Both techniques are more sensitive than the latex agglutination technique which has a cut-off at the 1:80 dilution of antigen. A direct instrumented comparison of the two techniques is presented below.

**Table 3**

| FOLD ANTIGEN DILUTION | mVOLTS/LATEX | mVOLTS/ENZYME |
|---|---|---|
| 0 | 3.0 | 11.0 |
| 1:320 | 32.0 | 203.0 |
| 1:160 | 63.0 | 290.0 |
| 1:80 | 113.0 | 272.0 |
| 1:40 | 195.0 | 194.0 |
| 1:20 | 316.0 | 168.0 |
| 1:10 | 428.0 | 258.0 |

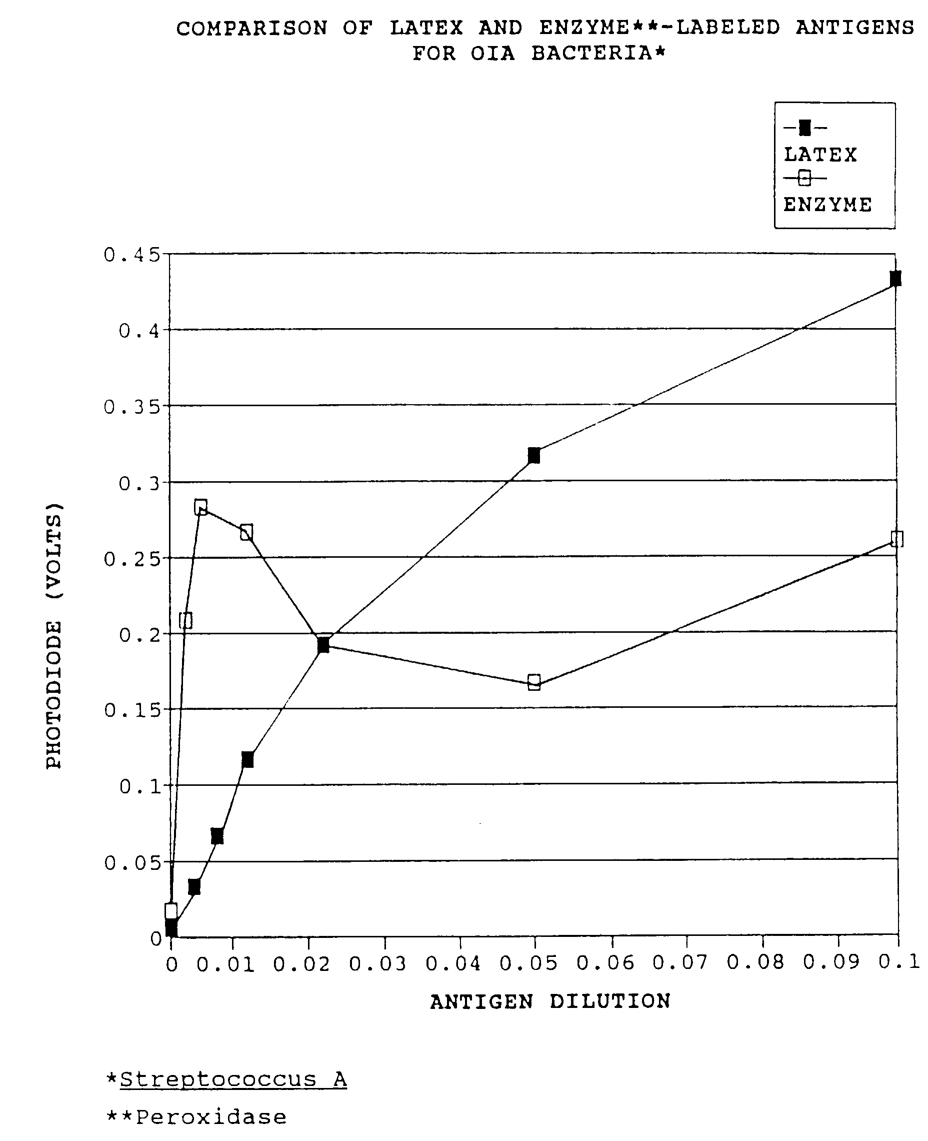

The above graph shows that, while the enzyme system in this assay tends to saturate more rapidly than the latex system, this is a qualitative testing system and the maximum generation of signal at the cut-off level of sensitivity is the desired result. The overall performance of the enzyme method improves the Streptococcus A - OIA dramatically.

### EXAMPLE 4

### Multiple Test Procedures

Using the procedures above-described, several peroxidase-antibody conjugates are combined in one reagent (about 3 drops). This is combined with an equal volume (75µl) of CSF sample and then mixed before pipetting one drop (25µl) successively onto the five antibody spots which have appropriate specifities. Incubation follows for about 2 minutes, after which the wafer is washed and dried. Incubation for 5 minutes then follows with one drop (25µl) added to each specific antibody spot. After this time period, and before reading, the wafer is washed and dried. In this manner, a single sample is easily analyzed for the presence of one or more analyte. In no case did a visible response occur in an antibody zone which corresponded to an antigen absent from the sample. Positive responses to added antigens generated signals comparable to the signal generated in a single test procedure.

### EXAMPLE 5

Comparison of Optical Immunoassay (OIA) and Enzyme-Linked Immunoadsorbant Assay (ELISA) techniques show the performance of the enzyme amplified OIA for Meningitidis A,C,Y W₁₃₅ relative to the ELISA using TMB as a substrate.

Identical experiments were run using OIA and ELISA techniques. These experiments demonstrate the advantages in sensitivity and readability obtained with OIA over ELISA as shown in the following Tables 4, 5 and 6 and the accompanying graphs for each table.

There are two major differences between these techniques. First, OIA utilizes a polished silicon wafer for solid phase adsorption of antibody while ELISA utilizes a clear polystyrene microtiter plate. Second, and more important, the substrates used to develop the reaction for OIA produce an insoluble product that deposits on the surface of the polished silicon wafer, while the substrate for ELISA produces a colored solution in the wells of the microtiter plate. It is because of this important difference between the results obtained that OIA is more effective.

Depositing mass on a reflective surface changes the refractive index of the light passing through the mass compared as to its surroundings. Therefore, as a visual or optical assay, the light reflected back is perceived as a color change, but the technique is not reliant on a dye system. The ELISA technique is solely reliant on a dye system and the solution in the microtiter wells must change color, otherwise the reaction is undetectable.

### Surface Preparation

One surface was a polished silicon wafer (OIA) and the other surface, a clear polystyrene, microtiter plate (ELISA).

Both surfaces received a 10 ug/ml antibody solution for 1 hour at room temperature, a deionized water rinse, a 0.5 mg/ml casein blocking solution for 10 minutes at room temperature, and a final deionized water rinse.

### Conducting The Assay

| | |
|---|---|
| Antigen Dilutions: | 1:5,000 |
| | 1:10,000 |
| | 1:20,000 |
| | 1:40,000 |
| | 1:80,000 |
| | 1:160,000 |
| | 0 |

| | |
|---|---|
| Conjugate Solution: | 1:100 dilution of HRP labeled antibody |
| | 5 mg/ml casein |
| | 50 mM MOPSO pH 7.0 |

One part of each antigen dilution was combined with one part conjugate solution immediately before use and applied to each surface. This was allowed to react for 2 minutes at room temperature, then each surface was rinsed with deionized water.

Substrate was then added to each surface. The OIA silicon wafer received TMBlue and the ELISA plate received TMB. This was allowed to react for 5 minutes at room temperature.

At this point, the reaction was over and the silicon wafer was rinsed with deionized water and dried with nitrogen. A visual reading was made to determine the furthest antigen dilution differentiable from the negative and the sample containing the insoluble product deposited on the surface put into the ellipsometer to measure the respective voltages.

The ELISA plate was also read visually while still reacting then the reaction was stopped with an acid solution and read again. The stopped reaction plate was put in the spectrophotometer to determine the optical densities.

The results obtained are as follows.

### Visually

OIA can be read out to a 1:40,000 antigen dilution as compared to ELISA unstopped can be read to only a 1:10,000-1:20,000 dilution while stopped can only be read to a 1:5,000-1:10,000 dilution.

### Instrument Read Results

**Table 4**

| Input Data | | | |
|---|---|---|---|
| X-VALUE | BACKGROUND | FOREGROUND | Y-VALUE |
| 5,000 | 0.3802 | 0.7010 | 0.3208 |
| 5,000 | 0.3802 | 0.7374 | 0.3572 |
| 10,000 | 0.3802 | 0.5198 | 0.1396 |
| 10,000 | 0.3802 | 0.5492 | 0.1690 |
| 20,000 | 0.3802 | 0.4340 | 0.0538 |
| 20,000 | 0.3802 | 0.4442 | 0.0640 |
| 40,000 | 0.3802 | 0.3985 | 0.0184 |
| 40,000 | 0.3802 | 0.4080 | 0.0278 |
| 80,000 | 0.3802 | 0.3913 | 0.0111 |
| 80,000 | 0.3802 | 0.3960 | 0.0158 |
| 160,000 | 0.3802 | 0.3879 | 0.0077 |
| 160,000 | 0.3802 | 0.3941 | 0.0139 |
| 0.000 | 0.3802 | 0.3865 | 0.0063 |
| 0.000 | 0.3802 | 0.3860 | 0.0058 |

| OBS | X-VALUE | AVG-Y | SD(N-1) | CV(%) | Y -/+ 1 SD | | Y -/+ 1 SD | |
|---|---|---|---|---|---|---|---|---|
| 2 | 0.000 | 0.006 | 0.000 | 5.4 | 0.01 | 0.01 | 0.01 | 0.01 |
| 2 | 5,000 | 0.339 | 0.026 | 7.6 | 0.31 | 0.36 | 0.29 | 0.39 |
| 2 | 10,000 | 0.154 | 0.021 | 13.5 | 0.13 | 0.18 | 0.11 | 0.20 |
| 2 | 20,000 | 0.059 | 0.007 | 12.2 | 0.05 | 0.07 | 0.04 | 0.07 |
| 2 | 40,000 | 0.023 | 0.007 | 29.0 | 0.02 | 0.03 | 0.01 | 0.04 |
| 2 | 80,000 | 0.013 | 0.003 | 24.8 | 0.01 | 0.02 | 0.01 | 0.02 |
| 2 | 160,000 | 0.011 | 0.004 | 40.6 | 0.01 | 0.02 | 0.00 | 0.02 |

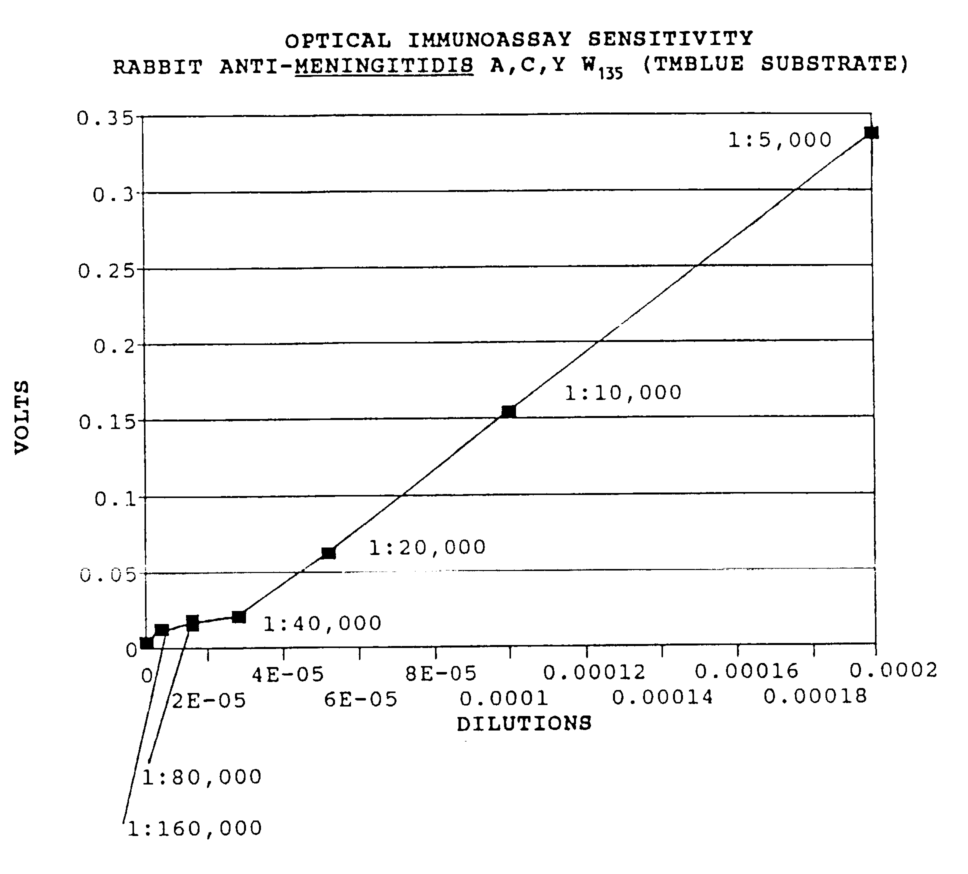

**Table 5**

| DILUTION | OPTICAL DENSITY READINGS AT 450 nM | | | |
|---|---|---|---|---|
| | 1 | 2 | 3 | 4 |
| 0 | 0 | 0 | 0.013 | 0.003 |
| 1:160,000 | 0 | 0 | 0.008 | 0.015 |
| 1:80,000 | 0.006 | 0.036 | 0.037 | 0.045 |
| 1:40,000 | 0.006 | 0.005 | 0.027 | 0.001 |
| 1:20,000 | 0.015 | 0.012 | 0.012 | 0.041 |
| 1:10,000 | 0.030 | 0.043 | 0.068 | 0.053 |
| 1:5,000 | 0.081 | 0.085 | 0.097 | 0.123 |
| 1:5,000 | 0.063 | 0.064 | 0.094 | 0.088 |

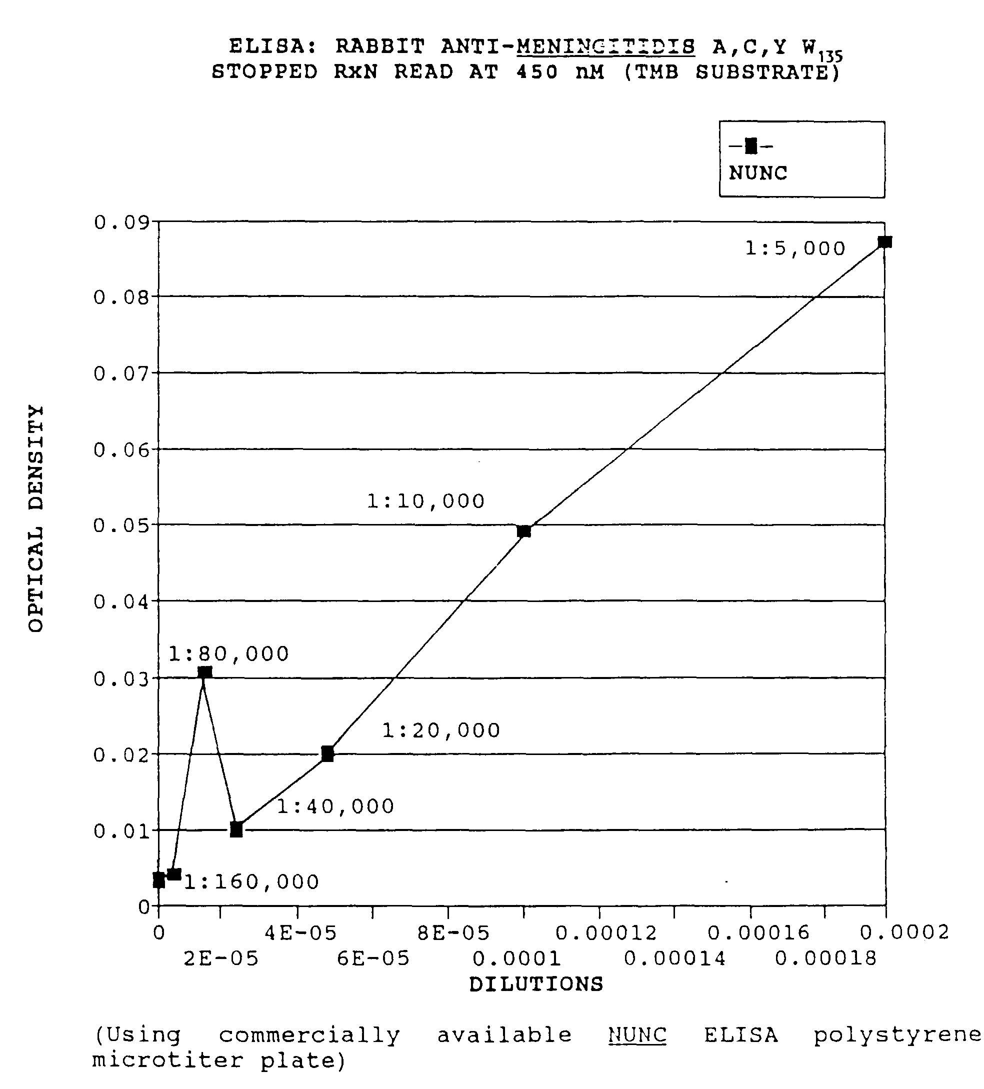

**Table 6**

| DILUTION | OPTICAL DENSITY READINGS AT 450 nM | | | |
|---|---|---|---|---|
| | 1 | 2 | 3 | 4 |
| 0 | 0 | 0 | 0 | 0 |
| 1:160,000 | 0 | 0 | 0 | 0 |
| 1:80,000 | 0.022 | 0.010 | 0.011 | 0.020 |
| 1:40,000 | 0.030 | 0.023 | 0.045 | 0.050 |
| 1:20,000 | 0.028 | 0.062 | 0.038 | 0.031 |
| 1:10,000 | 0.035 | 0.039 | 0.040 | 0.040 |
| 1:5,000 | 0.057 | 0.062 | 0.072 | 0.086 |
| 1:5,000 | 0.040 | 0.048 | 0.070 | 0.070 |

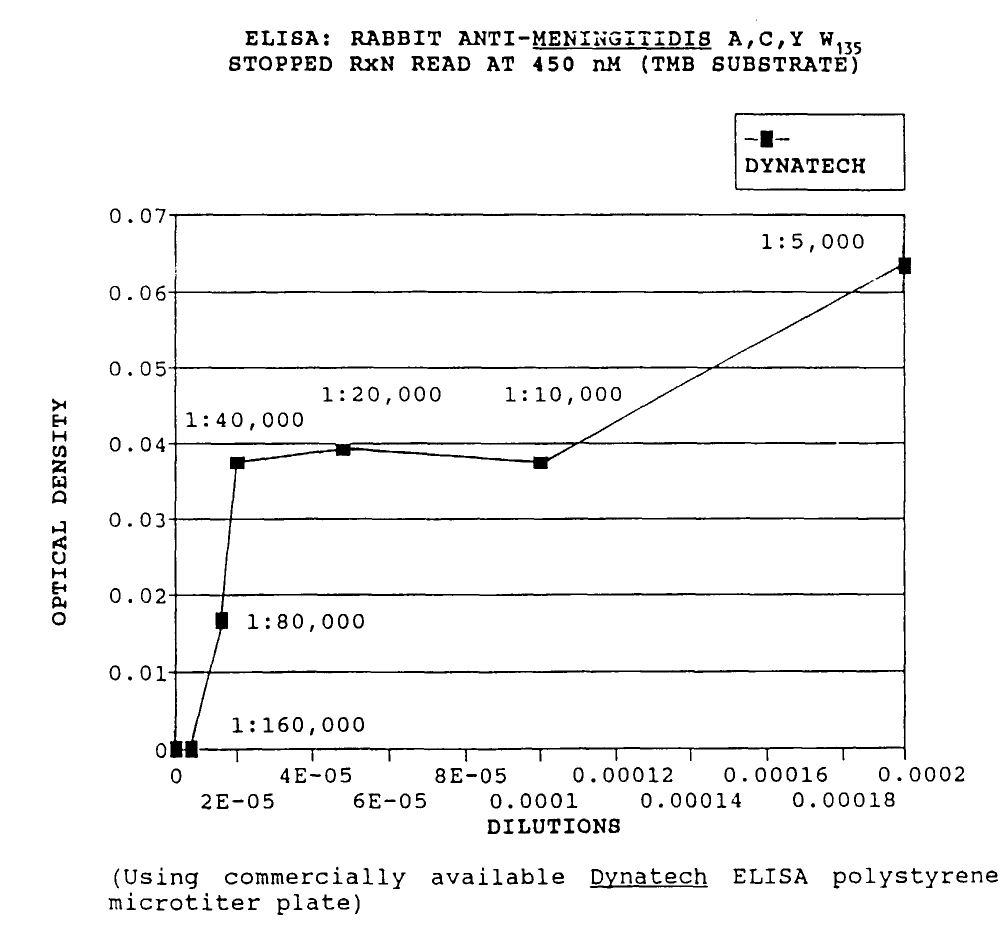

The following terms are defined for the above.
- OBS:: The number of measurements made at a given value of X.
- X-Value:: Concentration tested; known
- Y-Value:: Photodiode reading in volts of a reacted area given in terms of Foreground-Background; unknown.
- Avg-Y:: The mean value of Y (volts) measured by the photodiode; i.e., Y₁ + Y₂ + ... Y_{N}/N; where N=OBS.
- S.D. (n-1):: The standard deviation, calculated with the N-1 formula, where N = OBS, in the mean value of Y.
- CV (%):: The coefficient of variance is calculated from the Y values and expressed as a percentage. CV - S.D. divided by the mean value of Y.
- Y +/- 1 S.D.:: Calculates the range of possible Y values; range of possible Y values expressed as Mean + 1 x S.D. to Mean - 1 x S.D.
- Y +/- 2 S.D.:: Calculates the range of possible Y values; range of possible Y values expressed as Mean + 2 x S.D. to Mean - 2 x S.D.
- Background:: Intensity reading in volts of the unreacted antibody coated testpiece.
- Foreground:: Intensity reading in volts of the reacted zone of the testpiece.

In the table, the X-Value supplied is the following:

| | |
|---|---|
| 0 | The negative Control; buffer only |
| 5 | Represents a 1:5,000 dilution of a stock antigen preparation |
| 10 | Represents a 1:10,000 dilution of a stock antigen preparation |
| 20 | Represents a 1:20,000 dilution of a stock antigen preparation |
| 40 | Represents a 1:40,000 dilution of a stock antigen preparation |
| 80 | Represents a 1:80,000 dilution of a stock antigen preparation |
| 160 | Represents a 1:160,000 dilution of a stock antigen preparation |

The X-value designations given are used, as the software package in the photodiode data acquisition mode expects X-values to be concentrations in terms of ng/ml not a fold dilution. All of the Y-values, in volts, are collected relative to a known X-value and are listed in terms of that supplied value. The antigen preparations used throughout the work were produced as follows: the bacteria were grown anaerobically in Todd-Hewitt Broth for 24-48 hours at 37°C, the fluid was centrifuged to deposit the cells, the cells were washed in buffer and re-centrifuged (2x), the pellet of cells was resuspended in buffer and heat inactivated or treated with formalin. The inactivated and lysed cell preparation was filtered to produce a cell-free filtrate of antigen derived from the original organism, hereafter designated stock antigen. The stock antigen was diluted with 50 mM MOPS, pH=7.0 to produce standards for use in evaluation of the test performance.

As the result of a positive reaction, the photodiode modification of the Comparison ellipsometer allows the optical thickness change that occurs to be perceived as a change in light intensity which is recorded as a change in voltage at the detector. The voltage is a direct function of a change in intensity of the light collected by the photodiode and is proportional to the increase in optical thickness generated by interaction of the analyte/secondary reagent complex with the immobilized ligand on the surface of the slide. The method relies on the fact that when the reference and the test surface are identical in optical thickness (thickness multiplied by the refractive index), no light is perceived at the detector and a true zero (0 volts) would be observed. As there is a slight mismatch between the reference standard and the testpiece, the background of a testpiece is never a true zero. This is easily compensated for by measuring the background of an unreacted zone of the testpiece and subtracting this value from the intensity of a reacted zone of the testpiece. The reacted zone may be due to a positive or negative control or sample. As we know, from the concentration of the antigen applied to the surface in these examples, the observed value can be related to a specific concentration. Measurements are performed using a negative control; this is effectively a blank for this system and if desired the residual signal generated from the negative may also be subtracted from the positive sample. Thickness changes are not measured in terms of a discrete physical measurement such as Angstroms.

The enzyme-labeled antibody is not coated on to the surface of the wafer. Only when the antigen is present in a sample does the enzyme-labeled reagent have a chance to interact with the ligand on the surface of the wafer and then only through the antigen. The antigen must serve as the filling in the sandwich between immobilized ligand or receptive material and the labeled secondary reagent. In the specific examples, the ligand and the secondary reagent are both antibodies. The antigen/labeled antibody complex is captured by the immobilized ligand and forms a new layer which is a composite of layer 5 and 6. Layers 5 and 6 do not form as discrete layers, but rather form simultaneously.

The formed complex exists between the antigen in the analyte of interest and two antibodies. One of the antibodies being bound to the insoluble support as a receptive mediate layer thereon and the other antibody being a second unbound labeled antibody. A portion of this labeled antibody becomes bound to the support through the complex formed and with the receptive material and when it is precipitated, and represents the measured change indicating the presence of the analyte of interest.

### EXAMPLE 7

Nitrocellulose membranes coated with anti-Streptococcus A antibody, included in a Becton Dickinson commercially available test kit, were reacted with dilutions of the positive Streptococcus A specific antigen preparation used in Example 4. The antigen standards were mixed 1:1 with a 1:100 dilution of the anti-Streptococcus A antibody/HRP conjugate with 20 mg/ml casein in 50 mM MOPS, pH=7.0. 80 microliters of sample was applied to the membrane and allowed to incubate for 2 minutes as in the Streptococcus A - OIA enzyme assay. The membranes were then rinsed with water and 250 µl of TM Blue applied and allowed to react for 5 minutes. The membranes were rinsed again and a visual interpretation of the assay sensitivity made. A 1:40 dilution of the antigen gave a clear triangle as the manufacturer's test is designed to do in a positive reaction. A very weak triangle was formed with a 1:80 dilution of the standard. This compared to a 1:1600 dilution producing a visible response in the Streptococcus A - OIA, enzyme amplified sample of the present invention.

### EXAMPLE 8

Nitrocellulose membranes from Millipore were coated with anti-Streptococcus A polyclonal antibody using the exact protocol used for coating of the OIA wafer. These membranes were tested in the protocol used above, except that the sample volumes used were 30 µl of sample and 30 µl of TMBlue. As observed with the Becton Dickinson membranes, a visually positive result relative to the negative is only observed with a 1:40 dilution of the antigen preparation. A clear improvement in the sensitivity is achieved by the devices of the present invention coupling the use of the thin film optical detection scheme and the precipitating reagent TM Blue.

This example demonstrates that the devices of the present invention are up to at least 40 times more sensitive in exhibiting the signals evidencing the presence of very low concentrations of an analyte of interest than devices of the prior art which are neither HRP complexes and precipitating agent combinations nor are non-HRP combinations such as alkaline phosphatase.

## Claims

1. Method for detecting an analyte of interest in a sample, comprising the steps of:
(a) providing a thin film optical immunoassay device comprising a substrate which is non-polymeric, non-particulate and non-latex, having an upper surface able to generate a signal and a lower surface, and supporting on its upper surface, a receptive material able to specifically bind with said analyte of interest, adding to said device the analyte of interest and an enzyme labelled secondary receptive material able to specifically bind with said analyte of interest;
(b) contacting said enzyme-labeled secondary receptive material with a precipitating agent for a time period sufficient to cause precipitation of product from interaction of said precipitating agent and said enzyme; and
(c) measuring the increase in optical thickness on the substrate of the enzyme-labeled secondary receptive material layer and the unlabelled receptive material layer, said increase being an indication of the amount of said analyte in said test sample.

2. The method of claim 1, wherein said enzyme-labeled secondary receptive material comprises an antibacterial-antibody-enzyme complex.

3. The method of claim 1, wherein said enzyme-labeled secondary receptive material comprises alkaline phosphatase.

4. The method of claim 1, wherein the analyte of interest is selected from the group consisting of an antibody, antigen, allergens, enzymes, enzyme substrates, coenzymes, hormones, hormone receptors, proteins, blood proteins, tissue proteins, cells, cellular debris, nuclear material, viruses, viral particles, metabolites, neuron transmitters, haptens, drugs, nucleic acid, metals, microorganisms, parasites and bacteria.

5. The method of claim 1, wherein the analyte of interest is the Strep Group A or B antigen.

6. The method of claim 1, wherein the said analyte of interest is or is derived from the causative organisms for meningitis, Neisseria meningitides, Streptococcus pneumonia, Streptococcus Group B, and Haemophilus influenza type B.

7. The method of claim 1, wherein said mass change is detected by the use of an instrument.

8. The method of claim 7, wherein said instrument is selected from the group consisting of an ellipsometer and a comparison ellipsometer.

9. The method of claim 1, wherein said mass change is detected by visual means.

10. The method of claim 1, wherein said substrate supports an anti-reflective layer.

11. The method of claim 1, wherein said upper substrate surface comprises an anti-reflective layer.

12. An thin film optical assay kit suitable for use in a method as claimed in claim 1 for detecting an analyte of interest, said kit comprising, a substrate which is non-polymeric, non-particulate and non-latex having an upper surface able to generate a signal and a lower surface and, supporting on its upper surface, a receptive material able to specifically bind with said analyte of interest, an enzyme-labelled secondary receptive material able to specifically bind said analyte, and a precipitating agent that upon contact with said enzyme-labelled secondary receptive material causes precipitation of a product which along with said enzyme-labelled secondary receptive material results in an increase in optical thickness of said analyte bound material and the receptive material.

13. The kit of claim 12, wherein the optical thickness is detected by the use of an instrument.

14. The kit of claim 13, wherein said instrument is selected from the group consisting of an ellipsometer and a comparison ellipsometer.

15. The kit of claim 12, wherein said optical thickness is detected by visual means.

16. The kit of claim 12, said enzyme-labelled secondary receptive material comprises an anti-bacterial-antibody-enzyme complex.

17. The kit of claim 12, wherein said enzyme-labelled secondary receptive material comprises alkaline phosphatase.

18. The kit of claim 12, wherein the analyte of interest is selected from the group consisting of an antibody, antigen, allergens, enzymes, enzyme substrates, coenzymes, hormones, hormone receptors, proteins, blood proteins, tissue proteins, cells, cellular debris, nuclear material, viruses, viral particles, metabolites, neuron transmitters, haptens, drugs, nucleic acid, metals, microorganisms, parasites and bacteria.

19. The kit of claim 12, wherein the analyte of interest is the Strep Group A or B antigen.

20. The kit of claim 12, wherein said analyte of interest is or is derived from the causative organisms for meningitis, Neisseria meningitides, Streptococcus pneumonia, Streptococcus Group B, and Hemophilus influenza type B.

21. The kit of claim 12, wherein said substrate supports an anti-reflective layer.

22. The kit of claim 12, wherein said upper substrate surface comprises an anti-reflective layer.

## Patentansprüche

1. Verfahren zum Nachweis eines Analyten von Interesse in einer Probe, umfassend die Schritte:
(a) Vorsehen einer optischen Dünnfilm-Immunoassayvorrichtung, umfassend ein Substrat, welches nicht-polymer, nicht-teilchenförmig und nicht Latex ist, mit einer oberen Oberfläche, die zur Erzeugung eines Signals in der Lage ist, und einer unteren Oberfläche, und wobei seine obere Oberfläche ein aufnahmefähiges Material trägt, das in der Lage ist, spezifisch mit dem Analyten von Interesse zu binden, Hinzugeben des Analyten von Interesse und eines Enzym-markierten sekundären aufnahmefähigen Materials, das in der Lage ist, spezifisch den Analyten von Interesse zu binden, zur Vorrichtung;
(b) Kontaktieren des Enzym-markierten sekundären aufnahmefähigen Materials mit einem Präzipitierungsmittel während einer Zeitdauer, die ausreicht, um eine Präzipitation eines aus der Wechselwirkung des Präzipitierungsmittels und des Enzyms resultierenden Produkts zu bewirken; und
(c) Messen der Zunahme der optischen Dicke auf dem Substrat der Enzym-markierten sekundären aufnahmefähigen Materialschicht und der unmarkierten aufnahmefähigen Materialschicht, wobei die Zunahme ein Hinweis auf die Menge des in der Testprobe vorliegenden Analyten darstellt.

2. Verfahren gemäß Anspruch 1, wobei das Enzym-markierte sekundäre aufnahmefähige Material einen Anti-Bakterien-Antikörper-Enzym-Komplex umfaßt.

3. Verfahren gemäß Anspruch 1, wobei das Enzym-markierte sekundäre aufnahmefahige Material Alkalische Phosphatase umfaßt.

4. Verfahren gemäß Anspruch 1, wobei der Analyt von Interesse gewählt wird aus der einen Antikörper, ein Antigen, Allergene, Enzyme, Enzymsubstrate, Coenzyme, Hormone, Hormonrezeptoren, Proteine, Blutproteine, Gewebeproteine, Zellen, Zelltrümmer, Kernmaterial, Viren, virale Partikel, Metabolite, Neurotransmitter, Haptene, Arzneimittel, Nucleinsäure, Metalle, Mikroorganismen, Parasiten und Bakterien umfassenden Gruppe.

5. Verfahren gemäß Anspruch 1, wobei der Analyt von Interesse ein Antigen der Strep-Gruppe A oder B ist.

6. Verfahren gemäß Anspruch 1, wobei der Analyt von Interesse abgeleitet ist von Meningitis verursachenden Organismen, *Neisseria meningitides, Streptococcus pneumonia, Streptococcus* Gruppe B und *Haemophilus influenza* Typ B oder selbst ist.

7. Verfahren gemäß Anspruch 1, wobei die Massenänderung durch Verwendung eines Instrumentes nachgewiesen wird.

8. Verfahren gemäß Anspruch 7, wobei das Instrument ausgewählt wird aus der ein Ellipsometer und ein Vergleichs-Ellipsometer umfassenden Gruppe.

9. Verfahren gemäß Anspruch 1, wobei die Massenänderung durch visuelle Mittel nachgewiesen wird.

10. Verfahren gemäß Anspruch 1, wobei das Substrat eine anti-reflektive Schicht trägt.

11. Verfahren gemäß Anspruch 1, wobei die obere Oberfläche des Substrates eine anti-reflektive Schicht beinhaltet.

12. Optischer Dünnfilm-Assaykit, geeignet zur Verwendung bei einem Verfahren gemäß Anspruch 1 zum Nachweis eines Analyten von Interesse, wobei das Kit ein Substrat, welches nicht-polymer, nicht-teilchenförmig und nicht Latex ist, mit einer oberen Oberfläche, die zur Erzeugung eines Signals in der Lage ist, und einer unteren Oberfläche, und wobei seine obere Oberfläche ein aufnahmefähiges Material trägt, das in der Lage ist, spezifisch mit dem Analyten von Interesse zu binden, ein Enzym-markiertes sekundäres aufnahmefähiges Material, das in der Lage ist, spezifisch den Analyten von Interesse zu binden, und ein Präzipitierungsmittel, welches beim Kontakt mit dem Enzym-markierten sekundären aufnahmefähigen Material zur Präzipitation eines Produkts führt, was zusammen mit dem Enzym-markierten sekundären aufnahmefähigen Material zu einer Zunahme der optischen Dicke des Analyt-gebundenen Materials und des aufnahmefähigen Materials führt, umfaßt.

13. Kit gemäß Anspruch 12, wobei die optische Dicke durch die Verwendung eines Instrumentes nachgewiesen wird.

14. Kit gemäß Anspruch 13, wobei das Instrument ausgewählt wird aus der ein Ellipsometer und ein Vergleichs-Ellipsometer umfassenden Gruppe.

15. Kit gemäß Anspruch 12, wobei die optische Dicke durch visuelle Mittel bestimmt wird.

16. Kit gemäß Anspruch 12, wobei das Enzym-markierte sekundäre aufnahmefähige Material einen Anti-Bakterien-Antikörper-Enzym-Komplex umfaßt.

17. Kit gemäß Anspruch 12, wobei das Enzym-markierte sekundäre aufnahmefähige Material Alkalische Phosphatase umfaßt.

18. Kit gemäß Anspruch 12, wobei der Analyt von Interesse gewählt wird aus der einen Antikörper, ein Antigen, Allergene, Enzyme, Enzymsubstrate, Coenzyme, Hormone, Hormonrezeptoren, Proteine, Blutproteine, Gewebeproteine, Zellen, Zelltrümmer, Kernmaterial, Viren, virale Partikel, Metabolite, Neurotransmitter, Haptene, Arzneimittel, Nucleinsäure, Metalle, Mikroorganismen, Parasiten und Bakterien umfassenden Gruppe.

19. Kit gemäß Anspruch 12, wobei der Analyt von Interesse ein Antigen der Strep-Gruppe A oder B ist.

20. Kit gemäß Anspruch 12, wobei der Analyt von Interesse abgeleitet ist von Meningitis verursachenden Organismen, *Neisseria meningitides, Streptococcus pneumonia, Streptococcus* Gruppe B und *Haemophilus influenza* Typ B oder selbst ist.

21. Kit gemäß Anspruch 12, wobei das Substrat eine anti-reflektive Schicht trägt.

22. Kit gemäß Anspruch 12, wobei die obere Oberfläche des Substrates eine anti-reflektive Schicht beinhaltet.

## Revendications

1. Méthode pour détecter un analyte d'intérêt dans un échantillon, comprenant les étapes consistant :
(a) à fournir un dispositif de test immunologique optique à film mince comprenant un substrat qui est non polymèrique, non particulaire et non latex, ayant une surface supérieure capable de générer un signal et une surface inférieure, et supportant sur sa surface supérieure, un matériel récepteur capable de se lier spécifiquement avec ledit analyte d'intérêt, à ajouter audit dispositif l'analyte d'intérêt et un matériel récepteur secondaire marqué par une enzyme capable de se lier spécifiquement avec ledit analyte d'intérêt ;
(b) à mettre en contact ledit matériel récepteur secondaire marqué par une enzyme avec un agent de précipitation pendant une période de temps suffisante pour provoquer la précipitation du produit provenant de 1' interaction dudit agent de précipitation et de ladite enzyme ; et
(c) à mesurer l'augmentation de l'épaisseur optique sur le substrat de la couche de matériel récepteur secondaire marqué par une enzyme et de la couche de matériel récepteur non marqué, ladite augmentation étant une indication de la quantité dudit analyte dans ledit échantillon de test.

2. Méthode de la revendication 1, dans laquelle ledit matériel récepteur secondaire marqué par une enzyme comprend un complexe enzyme-anticorps antibactérien.

3. Méthode de la revendication 1, dans laquelle ledit matériel récepteur secondaire marqué par une enzyme comprend de la phosphatase alcaline.

4. Méthode de la revendication 1, dans laquelle ledit analyte d'intérêt est choisi dans le groupe constitué par un anticorps, antigène, allergènes, enzymes, substrats enzymatiques, coenzymes, hormones, récepteurs hormonaux, protéines, protéines du sang, protéines tissulaires, cellules, débris cellulaires, matériel nucléaire, virus, particules virales, métabolites, neurotransmetteurs, haptènes, médicaments, acide nucléique, métaux, micro-organismes, parasites et bactéries.

5. Méthode de la revendication 1, dans laquelle l'analyte d'intérêt est l'antigène Strep Groupe A ou B.

6. Méthode de la revendication 1, dans laquelle ledit analyte d'intérêt est ou est dérivé des organismes provoquant la méningite, Neisseria meningitidis, Streptococcus pneumoniae, Streptococcus Groupe B, et Haemophilus influenza type B.

7. Méthode de la revendication 1, dans laquelle ledit changement de masse est détecté en utilisant un instrument.

8. Méthode de la revendication 7, dans laquelle ledit instrument est choisi dans le groupe constitué par un ellipsomètre et un ellipsomètre de comparaison.

9. Méthode de la revendication 1, dans laquelle ledit changement de masse est détecté par des moyens visuels.

10. Méthode de la revendication 1, dans laquelle ledit substrat supporte une couche anti-réflexion.

11. Méthode de la revendication 1, dans laquelle ladite surface supérieure du substrat comprend une couche anti-réflexion.

12. Kit de tests optique à film mince approprié à l'utilisation dans une méthode telle que revendiquée dans la revendication 1 pour détecter un analyte d'intérêt, ledit kit comprenant un substrat qui est non polymèrique, non particulaire, et non latex, ayant une surface supérieure capable de générer un signal et une surface inférieure et, supportant sur sa surface supérieure, un matériel récepteur capable de se lier spécifiquement avec ledit analyte d'intérêt, un matériel récepteur secondaire marqué par une enzyme capable de se lier spécifiquement avec ledit analyte, et un agent de précipitation qui par contact avec ledit matériel récepteur secondaire marqué par une enzyme provoque la précipitation d'un produit qui avec ledit matériel récepteur secondaire marqué par une enzyme entraîne une augmentation de l'épaisseur optique dudit matériel lié à l'analyte et du matériel recepteur.

13. Kit de la revendication 12, dans lequel l'épaisseur optique est détectée en utilisant un instrument.

14. Kit de la revendication 13, dans lequel ledit instrument est choisi dans le groupe constitué par un ellipsomètre et un ellipsomètre de comparaison.

15. Kit de la revendication 12, dans lequel ladite épaisseur optique est détectée par des moyens visuels.

16. Kit de la revendication 12, dans lequel ledit récepteur secondaire marqué par une enzyme comprend un complexe enzyme-anticorps antibactérien.

17. Kit de la revendication 12, dans lequel ledit matériel récepteur secondaire marqué par une enzyme comprend de la phosphatase alcaline.

18. Kit de la revendication 12, dans lequel l'analyte d'intérêt est choisi dans le groupe constitué par un anticorps, antigène, allergènes, enzymes, substrats enzymatiques, coenzymes, hormones, récepteurs hormonaux, protéines, protéines du sang, protéines tissulaires, cellules, débris cellulaires, matériel nucléaire, virus, particules virales, métabolites, neurotransmetteurs, haptènes, médicaments, acide nucléique, métaux, micro-organismes, parasites et bactéries.

19. Kit de la revendication 12, dans lequel l'analyte d'intérêt est l'antigène Strep Groupe A ou B.

20. Kit de la revendication 12, dans lequel ledit analyte d'intérêt est ou est dérivé des organismes provoquant la méningite, Neisseria meningitidis, Streptococcus pneumoniae, Streptococcus Groupe B, et Haemophilus influenza type B.

21. Kit de la revendication 12, dans lequel ledit substrat supporte une couche anti-réflexion.

22. Kit de la revendication 12, dans lequel ladite surface supérieure du substrat comprend une couche anti-réflexion.
